# EUROPEAN PATENT APPLICATION

(11) **EP 3 848 085 A1**
(43) Date of publication of application: **14.07.2021**
(21) Application number: 19870950.3
(22) Date of filing: 27.09.2019
(51) Int. Cl.: A61M 37/00, A61K 9/00

(54) **MICROSTRUCTURE**

(30) Priority: 08.10.2018 KR 20180119640
(71) Applicant: Industry-Academic Cooperation Foundation, Yonsei University, Seoul 03711 (KR); Juvic Inc., Seoul 08389 (KR)
(72) Inventor: JUNG, Hyungil, Seoul 03719 (KR); YANG, Huisuk, Seoul 06578 (KR); KANG, Geonwoo, Seoul 08388 (KR)
(74) Representative: Fuchs Patentanwälte Partnerschaft mbB
(86) International application number: PCT/KR2019/012589
(87) International publication number: WO 2020/075997

(57) **Abstract**

A microstructure is provided. A microstructure according to an embodiment of the present invention comprises: a tip portion formed on a substrate and comprising a drug; and a separation portion which is formed between the substrate and the tip portion and, after the tip portion has been inserted into the skin, separates the substrate and the tip portion physically by means of an external force or chemically by means of a chemical material.

## Description

### [Technical Field]

The present invention relates to a microstructure, and more particularly, to a microstructure that is usable to deliver a drug into skin.

### [Background Art]

In general, oral administration in a tablet form or capsule form or needles are used to deliver a drug for treatment of diseases or beauty into a body. Recently, various microstructures including microneedles have been developed. Microstructures developed to date have been mainly used for drug delivery in vivo, blood collection, and detection of analytes in a body.

When a biodegradable microstructure is inserted into skin, the biodegradable microstructure is not dissolved immediately but is completely dissolved depending on a material used, taking from several minutes to tens of minutes. Therefore, a patch that can be fixed to the skin is used to prevent separation of the microstructure from the skin while the microstructure is inserted into the skin and dissolved in the skin.

However, the conventional micro-patch requires a different patch application time depending on a skin condition of a subject. In this case, side effects such as skin irritation and inflammation are caused to the subject to which the patch is to be applied as an attachment time of the micro-patch increases.

In addition, before the drug contained in the microstructure is absorbed into the skin, a portion of the drug contained in the microstructure is melted outside the skin and remains in the patch due to sweat or body temperature rise. Accordingly, an amount of the drug delivered to the skin is uneven, and thus, a purpose of quantitative delivery cannot be achieved.

Accordingly, there is an increasing need for a microstructure in which a long attachment time of the micro-patch, low efficiency of drug delivery, and unevenness of the drug delivery amount can be solved.

### [Disclosure]

### [Technical Problem]

The present invention is directed to providing a microstructure which is separatable from a substrate within a short time after being inserted into skin.

In addition, the present invention is directed to providing a microstructure which is firmly insertable into skin to enable accurate drug delivery and is not separated from the skin by an external force.

### [Technical Solution]

One aspect of the present invention provides a microstructure including a tip portion including a drug, and a separation portion which is formed between a substrate and the tip portion and, after the tip portion is inserted into skin, separates the substrate from the tip portion physically by an external force or chemically by a chemical material.

The microstructure may further include a base portion formed on one side of the substrate or the tip portion, in which the separation portion may be formed between the base portion and the tip portion or between the substrate and the base portion, and after the tip portion is inserted into the skin, may separate the base portion and the tip portion or the substrate and the base portion.

The separation portion may include at least one selected from among a biodegradable polymer and sugar.

A dissolution time of the separation portion may be less than five minutes to dissolve by the chemical material or the separation portion may be dissolved by the chemical material by 70% or more per unit volume within three minutes.

The chemical material may be introduced between the substrate and the skin or may be applied on the skin before the tip portion is inserted into the skin.

The chemical material may include at least one selected from among water, an aqueous solution, and an injectable ester, the aqueous solution may include at least one selected from among anhydrous or aqueous lower alcohol having one to four carbon atoms, ethyl acetate, chloroform, 1,3-butylene glycol, hexane, diethyl ether, and butyl acetate, the injectable ester may include at least one selected from among a water-insoluble agent, propylene glycol, polyethylene glycol, an oily component, and ethyl oleate, and an oil useable as the oily component may include at least one selected from among a vegetable oil, a mineral oil, a silicone oil, and a synthetic oil.

In the separation portion, a cross section of a central portion may be smaller than cross sections of both sides.

The separation portion may be broken by a force smaller than a coupling force with the tip portion or the substrate.

The separation portion may be broken by a force smaller than a force by which the tip portion is separated from the skin.

The external force may be applied to the separation portion in a horizontal direction or a vertical direction.

The separation portion may contain a biodegradable material.

The separation portion may be formed so that a cross section decreases.

The tip portion may have a candle shape of which a vertical cross section decreases in a direction toward a tip or may have a continuous curvature.

The substrate may include a protrusion having a predetermined thickness at a portion of the substrate corresponding to the separation portion and may be formed of a horizontal straight line, an inclined oblique line, or a center convex or concave curve in a cross-sectional view.

Another aspect of the present invention provides a microstructure including a base portion formed on a substrate and having a first cross section, a separation portion having a second cross section smaller than the first cross section of the base portion and formed on the base portion to extend in a direction away from the substrate, and a tip portion having a third cross section larger than the second cross section of the separation portion and formed on the separation portion to extend in the direction away from the substrate, in which outer surfaces of the base portion, the separation portion, and the tip portion have a curvature continuous in the direction away from the substrate.

An area of the base portion may decrease in the direction away from the substrate, the second cross section may be located at a central portion of the separation portion when viewed in the direction away from the substrate and may be the narrowest cross section of cross sections of the separation portion, the third cross section may have a cross section larger than that of one end surface of the separation portion far from the substrate and may be the widest cross section of cross sections of the tip portion when viewed in the direction away from the substrate, and the tip portion may be formed in a pointed upper end of which a cross section next to the third cross section of the tip portion is narrowed in the direction away from the substrate.

The base portion, the separation portion, and the tip portion may be integrally formed, or the base portion and the tip portion may be formed of different materials.

A diameter of the first cross section may be in a range of 10 to 1,000 µm, a diameter of the second cross section may be in a range of 5 to 500 µm, an extension length from the substrate to the second end surface may be in a range of 50 to 1,000 µm, a diameter of the third cross section may be in a range of 100 to 500 µm, a distance from the third cross section to an end of the tip portion may be in a range of 100 to 1,000 µm, and a total extension length from the substrate to the end of the tip portion may be in a range of 200 to 2,000 µm.

The tip portion may include a first volume region located at a first extension length from one end of the separation portion to the third cross section in the direction away from the substrate, a second volume region located at an extension length twice the first extension length from the third cross section, and a third volume region located above the second volume region, a combined volume of the first volume region and the second volume region may be 2 to 1000 times a volume of the third volume region, the tip portion may have a volume of 1.5 to 100 times a volume of the base portion and the separation portion, and a volume in a height range of an upper 60% in a total height direction from the base portion to the tip portion may be in a range of 60 to 90% of a total volume.

### [Advantageous Effects]

In a microstructure according to one embodiment of the present invention, when the microstructure is inserted into skin, a cross-sectional area of a tip portion inserted into a body is greater than a cross-sectional area of a separation portion. Accordingly, the tip portion having a large volume can remain in the skin in a state in which the separation portion of the microstructure is physically or chemically cut, a large amount of drugs can be delivered into the skin, and the tip portion can be firmly fixed to the skin without being removed in a state of being inserted into the skin.

In the microstructure according to one embodiment of the present invention, the separation portion physically or chemically separates the substrate from the tip portion containing the drug. Accordingly, the tip portion containing the drug may be separated within a short time, or the base portion together with the tip portion may be separated within a short time. Therefore, it is possible to shorten an attachment time of a micro-patch, reduce side effects such as skin irritation and inflammation, and improve stability and convenience of use.

In the microstructure according to one embodiment of the present invention, the attachment time of the micro-patch is shortened. Therefore, it is possible to prevent a decrease in a skin absorption rate of the drug due to sweat, heat generation, inability to be fixed with respect to the skin, or the like caused by a use for a long time. Moreover, the same drug absorption rate and efficacy can be provided for each subject to which the patch is to be applied, the drug can be delivered regardless of skin conditions, an application site, and an environment of the subject to which the patch is to be applied, and thus, it is possible to improve the supply of substances.

In the microstructure according to one embodiment of the present invention, there is no need to use a separate shooting device for injecting the drug into the skin, and thus, it is possible to maximize advantages of no pain and no irritation.

In the microstructure according to one embodiment of the present invention, the tip portion containing the drug is completely inserted into the skin. Therefore, the tip portion is not dissolved at outside of the skin, there is no residue of the micro-patch, all of the tip portion is absorbed into the skin, and thus, quantitative delivery of the drug can be realized.

In the microstructure according to one embodiment of the present invention, the drug in the tip portion does not remain on the micro-patch. Accordingly, the micro-patch of the present invention is hygienic compared to the conventional micro-patch, and thus, it is possible to minimize incidences of biohazard to the subject to which the patch is to be applied, biohazardous waste, and secondary damages thereof.

### [Description of Drawings]

FIG. 1 is a cross-sectional view of a microstructure according to a first embodiment of the present invention.
FIG. 2 is a cross-sectional view of a microstructure according to a second embodiment of the present invention.
FIG. 3 is a cross-sectional view of a microstructure according to a third embodiment of the present invention.
FIG. 4 is a cross-sectional view illustrating a state in which the microstructure of FIG. 1 is inserted into skin.
FIG. 5 is a cross-sectional view illustrating a state in which the microstructure of FIG. 2 is inserted into skin.
FIG. 6 is a cross-sectional view illustrating a state in which the microstructure of FIG. 3 is inserted into skin.
FIG. 7 is a cross-sectional view for describing a process of chemically separating a separation portion in FIG. 4.
FIG. 8 is a cross-sectional view for describing a process of chemically separating a separation portion in FIG. 6.
FIG. 9 is a cross-sectional view for describing a process of physically separating the separation portion in FIG. 4.
FIG. 10 is a cross-sectional view for describing a process of physically separating the separation portion in FIG. 6.
FIG. 11 is a cross-sectional view of a modification example of the microstructure according to the first embodiment of the present invention.
FIG. 12 is a cross-sectional view of a modification example of the microstructure according to the second embodiment of the present invention.
FIG. 13 is a cross-sectional view of a modification example of the microstructure according to a third embodiment of the present invention.

### [Modes of the Invention]

Hereinafter, embodiments of the present invention will be described in detail with reference to the accompanying drawings so that those of ordinary skill in the art may easily implement the present invention. The present invention may be implemented in various different forms and is not limited to the embodiments described herein. In the drawings, parts irrelevant to the descriptions may be omitted in order to clearly describe the present invention, and the same reference numerals are assigned to the same or similar components throughout the specification.

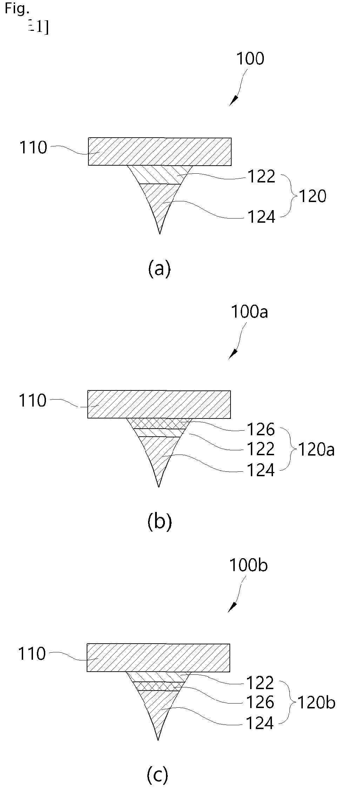

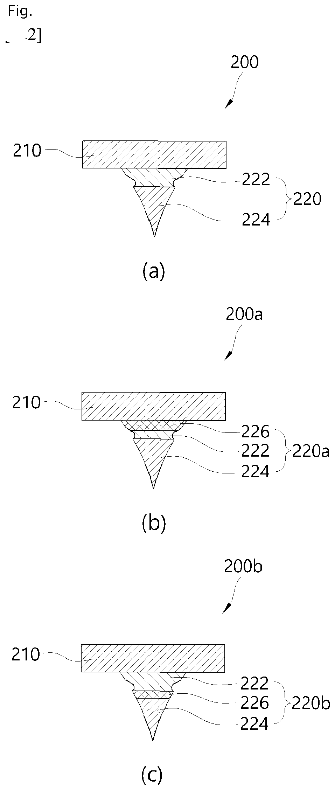

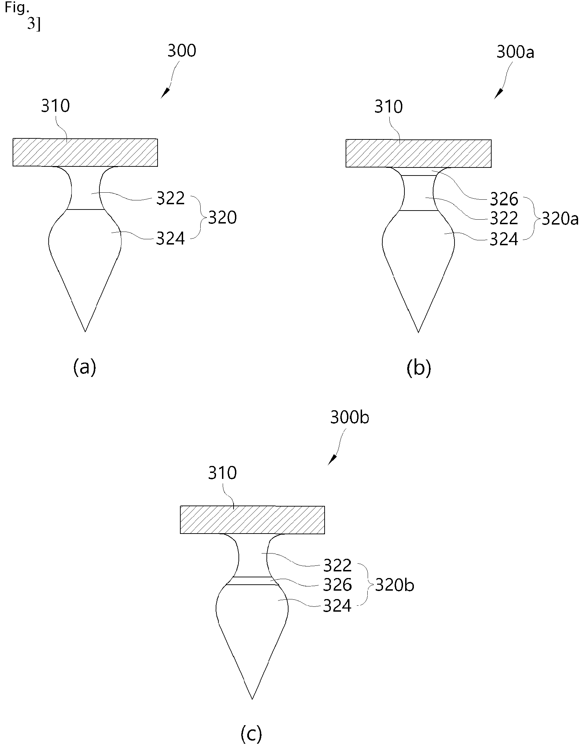

Hereinafter, microstructures according to the embodiments of the present invention will be described in more detail with reference to the drawings. FIG. 1 is a cross-sectional view of a microstructure according to a first embodiment of the present invention, FIG. 2 is a cross-sectional view of a microstructure according to a second embodiment of the present invention, and FIG. 3 is a cross-sectional view of a microstructure according to a third embodiment of the present invention.

Referring to FIG. 1, a microstructure 120 according to the first embodiment of the present invention may be formed in a substantially conical shape. The microstructure 120 includes a separation portion 122 and a tip portion 124. Here, a base portion 126 may be selectively formed.

As an example, as illustrated in FIG. 1B, in a microstructure 120a, the base portion 126 may be formed between a substrate 110 and the separation portion 122. As another example, as illustrated in FIG. 1C, in a microstructure 120b, the base portion 126 may be formed between the separation portion 122 and the tip portion 124.

Here, the separation portion 122, the tip portion 124, and the base portion 126 may be formed to have a cross-sectional area that decreases in a direction from the substrate 110 toward a tip of the tip portion 124 according to a formation order thereof. In particular, the tip portion 124 may have a cross-sectional area that decreases in a direction toward a tip thereof.

In this case, the microstructures 120, 120a, and 120b may be formed on the substrate 110 to form micro-patches 100, 100a, and 100b.

Referring to FIG. 2, a microstructure 220 according to the second embodiment of the present invention is similar to the microstructure 120 according to the first embodiment. However, in a separation portion 222 of the second microstructure 220, a cross section of a center portion may have an area smaller than those of cross sections of both sides. The microstructure 220 includes the separation portion 222 and a tip portion 224. Here, a base portion 226 may be selectively formed.

As an example, as illustrated in FIG. 2B, in a microstructure 220a, the base portion 226 may be formed between a substrate 210 and the separation portion 222. As another example, as illustrated in FIG. 2C, in a microstructure 220b, the base portion 226 may be formed between the separation portion 222 and the tip portion 224.

In this case, the microstructures 220, 220a, and 220b may be formed on the substrate 210 to form micro-patches 200, 200a, and 200b.

Referring to FIG. 3, a microstructure 320 according to the third embodiment of the present invention may be substantially candle shaped. That is, the microstructure 320 may be formed to have a continuous curvature in a vertical cross-sectional view. The microstructure 320 includes a separation portion 322 and a tip portion 324. Here, a base portion 326 may be selectively formed.

As an example, as illustrated in FIG. 3B, in a microstructure 320a, the base portion 326 may be formed between a substrate 310 and the separation portion 322. As another example, as illustrated in FIG. 3C, in a microstructure 320b, the base portion 326 may be formed between the separation portion 322 and the tip portion 324.

Here, the tip portion 324 may have a candle shape of which a cross-sectional area is increased by a predetermined distance from the separation portion 322 and decreased in a direction toward a tip.

In particular, in FIG. 3B, in the present embodiment, the base portion 326 has a first cross section in contact with the substrate, and a length of the first cross section, that is, a length of a diameter of the base portion 326 in contact with the substrate 310 may be in the range of 10 to 1,000 µm. However, the length of the diameter of the base portion 326 is not limited thereto.

In the present embodiment, the base portion 326 may be formed such that an area of a cross section decreases in a direction away from the substrate 310. An extension length of the base portion 326 in a direction away from the substrate 310 may be in the range of 10 to 500 µm but is not limited thereto. An upper surface of the base portion 326 may have a cross section smaller than the first cross section of the base portion 326, and in this case, a diameter of a cross section of the upper surface of the base portion 326 may be in the range of 5 to 750 µm.

As seen in FIG. 3B, the separation portion 322 is located above the base portion 326, that is, on an end of the base portion 326 in the direction away from the substrate 310.

The separation portion 322 may be formed to extend continuously or intermittently from the upper surface of the base portion 326 and may include a second end surface smaller than the first end surface of the base portion 326. The separation portion 322 may be formed in a cylindrical shape having a circular cross section.

In the present embodiment, the separation portion 322 may be formed to have a cross section having the narrowest area at a central portion of the separation portion 322 when viewed in the direction away from the substrate 310. In the present embodiment, the cross section, which is located at the central portion of the separation portion 322 and has the narrowest area among cross sections of the separation portion 322, is defined as the second cross section.

In the present embodiment, a diameter of the second cross section may be in the range of 5 to 500 µm.

In the present embodiment, the separation portion 322 may be symmetrically formed with respect to the second cross section when viewed in an extension direction of the separation portion 322, and this shape is formed by pulling the separation portion 322 by a centrifugal force or the like.

Meanwhile, an extension length from the substrate 310 to the second end surface of the separation portion 322 may be in the range of 50 to 1,000 µm, and the extension length of the separation portion 322 may be in the range of 50 to 1,000 µm.

In the present embodiment, a length of an upper end surface of the separation portion 322 may be formed to be equal to the diameter of the cross section of the upper surface of the base portion 326, and the tip portion 324 may be formed on the separation portion 322. The tip portion 324 may be connected to the separation portion 322 continuously or intermittently and may be formed in a columnar shape having a circular cross section extending from the separation portion 322.

The tip portion 324 has a third cross section having an area larger than that of the second cross section of the separation portion 322. In the present embodiment, the third cross section may be defined as a cross section having the largest area among cross sections forming the tip portion 324. In the present embodiment, a diameter of the third cross section may be in the range of 100 to 500 µm. In this case, a distance from the substrate to the third cross surface may be in the range of 100 to 1,000 µm, and a distance from a third cross section to a tip of the tip portion 324 may be in the range of 100 to 1,000 µm. In addition, an extension length of the base portion 326 may be in the range of 100 to 1,000 µm.

Accordingly, the cross section of the tip portion 324 is gradually widened in a direction from a lower portion having a narrower cross section than the third cross section toward the extension direction, and then the tip portion 324 has the widest cross section at the third cross section. Moreover, a cross section next to the third cross section of the tip portion 324 is gradually narrowed and is finished in a pointed shape at one end in the extension direction of the tip portion 324. That is, the tip portion 324 may have a candle shape of which a vertical cross section has a continuous curvature.

In the present embodiment, the tip portion 324 may include a first volume region located at a first extension length from one end of the separation portion 322 to the third cross section in the direction away from the substrate 310, a second volume region located at an extension length which is twice the first extension length from the third cross section, and a third volume region located above the second volume region. A length of an upper end surface of the second volume region may be smaller than or equal to a length of the upper end surface of the separation portion 322. In this case, a combined volume of the first volume region and the second volume region may be 2 to 1000 times a volume of the third volume region. In addition, the tip portion 324 may have a volume of 1.5 to 100 times those of the base portion 326 and the separation portion 322. In the present embodiment, the tip portion 324 may be a portion containing a drug which will be injected into the skin in a state in which the microstructure 320a is inserted into the skin, and as a size of the tip portion 324 increases, an amount of the drug injected into the skin may increase.

Accordingly, an entirety of the microstructure according to the present embodiment including the base portion 326, the separation portion 322, and the tip portion 324 is formed to have a candle shape.

In this case, outer surfaces of the base portion 326, the separation portion 322, and the tip portion 324 may be formed as a continuous curved surface in the direction away from the substrate 310. That is, the outer surfaces may have a continuous curvature in the direction away from the substrate 310. However, according to a manufacturing process of the base portion 326, the separation portion 322, and the tip portion 324, the base portion 326, the separation portion 322, and the tip portion 324 may be formed to have an intermittent surface.

In this case, the total extension length from the substrate 310 to the tip of the tip portion 324 of the microstructure may be in the range of 200 to 2,000 µm but is not limited thereto.

The microstructure 320a according to the present embodiment may be formed so that the inside is full and may be formed to contain the drug for administration to the skin therein.

Here, in the microstructure 320a according to the present embodiment, an insertion depth of the microstructure 320a inserted into the skin is changed according to a type of the drug to be inserted into the skin, a magnitude of force to insert the microstructure 320a into an endothelial layer of the skin to which the drug is to be administered, or the like. For example, the magnitude of the force may be 0.001 N or more.

Even when the microstructure 320a is inserted into the skin at one of various depths, in the microstructure 320a, the third cross section portion having the widest cross section of the tip portion 324 needs to be disposed at a position deeper than the epidermal layer of the skin.

When the third cross section portion having the widest cross section of the tip portion 324 does not pass through the epidermal layer of the skin and enter the skin, the microstructure 320a may not be embedded in the skin due to elasticity of the skin and may be separated from the skin to the outside of the skin.

In the microstructure 320a according to the present embodiment, in a state in which the portion having the widest cross section of the microstructure 320a, that is, the tip portion 324, is inserted into the skin by pressing the substrate 310 on which the microstructure 320a is formed inside the skin, by breaking the separation portion 322 of the microstructure 320a, the microstructure 320a may be located inside the skin.

In describing the microstructure according to the present embodiment, the microstructure is divided into the base portion, the separation portion 322 and a tip portion 324, and the length of one cross section of the base portion, the separation portion 322, and the tip portion 324 is described exemplarily. Although the microstructure is made of a single material and is integrally formed, the base portion, the separation portion 322, and the tip portion 324 may not be clearly partitioned. Accordingly, it will be readily understood by those skilled in the art that the length of the cross section is exemplary. In addition, depending on the manufacturing process, the base portion 326 may not be formed in the microstructure as illustrated in FIG. 3A.

Accordingly, by adjusting the lengths, widths, and cross-sectional areas of the tip portion 324, the separation portion 322, and the base portion 326, various skin insertion depths may be determined, the amount of drug to be administered may be adjusted, and thus, various microstructures may be provided.

In addition, a contact area between the tip portion 324 and the skin increases. Accordingly, the tip portion 324 by a body fluid may be dissolved faster, and a coupling force with the skin may increase. Therefore, the separation portion 322 may be broken by a relatively small physical force while the tip portion 324 is not separated outward from the skin. In addition, since the tip portion 324 has a convex shape in the central portion, an internal volume of the tip portion 324 increases, and thus, the amount of contained drug may increase.

In this case, the microstructures 320, 320a, and 320b may be formed on the substrate 310 to form micro-patches 300, 300a, and 300b.

However, in the present invention, the shape of the microstructure is not particularly limited thereto as long as it includes the separation portions 122, 222, and 322 for separating the tip portions 124, 224, and 324.

In the present invention, in the microstructure, the separation portions 122, 222, and 322 are separated by chemical dissolution of the separation portions 122, 222, and 322 through a solvent such as a chemical material or physical destruction of the separation portions 122, 222, and 322 by an external force, and thus it is possible to overcome limitations of the conventional micro-patch and improve accuracy, convenience, and safety of drug delivery.

In addition, in the microstructure, since the tip portions 124, 224, and 324 are separated within a short time after the tip portions 124, 224, and 324 are inserted into the skin, a supply of drugs to be delivered through the skin may be improved.

The drugs that are usable in the microstructure in the present invention are not particularly limited. For example, the drugs include chemical drugs, protein drugs, peptide drugs, nucleic acid molecules for gene therapy, nanoparticles, functional cosmetic active ingredients, or cosmetic ingredients.

In addition, for example, the drugs that are usable in the present invention include anti-inflammatory drug, analgesics, anti-arthritic drugs, antispasmodics, antidepressants, antipsychotic drugs, tranquilizers, anti-anxiety drugs, drug antagonists, anti-parkinosian drugs, cholinergic agonists, anticancer drugs, antiangiogenic drugs, immunosuppressants, antiviral drugs, antibiotics, appetite suppressants, pain relievers, anticholinergics, antihistamines, antimigraine drugs, hormones, coronary blood vessels, cerebrovascular or peripheral vasodilators, contraceptives, antithrombotic drugs, diuretics, antihypertensive agents, cardiovascular disease treatments, or cosmetic ingredients (for example, anti-wrinkle agents, skin aging inhibitors, and skin whitening agents) but are not limited thereto.

Here, the separation portions 122, 222, and 322, the tip portions 124, 224, and 324, and the base portions 126, 226, and 326 forming the microstructure may be integrally formed using the same material or may be formed of different materials.

The material forming the microstructure used in the present invention includes a biocompatible or biodegradable material. Here, the term "biocompatible material" refers to a material that is substantially non-toxic to a human body, is chemically inactive, and is not immunogenic. In the present specification, the term "biodegradable material" refers to a material that can be decomposed by body fluids, microorganisms, or the like in a living body.

Specifically, for example, biocompatible and/or biodegradable materials that are usable in the present invention are polyester, polyhydroxyalkanoate (PHAs), poly(α-hydroxyacid), poly(β-hydroxyacid), poly(3-hydrosicbutyrate-co-valerate; PHBV), poly(3-hydroxypropionate; PHP), poly(3-hydroxyhexanoate; PHH), poly(4-hydroxyacid), poly(4-hydroxybutyrate), poly(4-hydroxyvalerate), poly(4-hydroxyhexanoate), poly(esteramide), polycaprolactone, polylactide, polyglycolide, poly(lactide-co-glycolide; PLGA), polydioxanone, polyorthoester, polyetherester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazens, PHA-PEG, ethylene vinyl alcohol copolymer (EVOH), polyurethane, silicone, polyester, polyolefin, polyisobutylene and ethylene-alphaolefin copolymer, styrene-isobutylene-styrene triblock copolymers, acrylic polymers and copolymers, vinyl halide polymers and copolymers, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatics, polystyrene, polyvinyl ester, polyvinyl acetate, ethylene-methyl methacrylate copolymer, acrylonitrile-styrene copolymer, ABS resin and ethylenevinyl acetate copolymer, polyamide, alkyd resin, polyoxymethylene, polyimide, polyether, polyacrylate, polymethacrylate, polyacrylic acid-co-maleic acid, chitosan, dextran, cellulose, heparin, hyaluronic acid, alginate, inulin, starch, or glycogen, and preferably, are polyester, polyhydroxyalkanoate (PHAs), poly(α-hydroxy acid), poly(β-hydroxy acid), poly(3-hydrosoxybutyrate-co-valerate; PHBV), poly(3-hydroxypropionate; PHP), poly(3-hydroxyhexanoate; PHH), poly(4-hydroxyacid), poly(4-hydroxybutyrate), poly (4-hydroxyvalerate), poly(4-hydroxyhexanoate), poly(esteramide), polycaprolactone, polylactide, polyglycolide, poly(lactide-co-glycolide; PLGA), polydioxanone, polyorthoester, polyetherester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, chitosan, dextran, cellulose, heparin, hyaluronic acid, alginate, inulin, starch, or glycogen.

In this case, the microstructure may be formed by spotting viscous composition of drugs as described above. Here, the term "viscous composition" refers to a composition having ability to form a microstructure by changing the shape.

The separation portions 122, 222, and 322 are formed on the substrates 110, 210, and 310 (see FIGS. 1A, 1C, 2A, 2C, 3A, and 3C) or are formed on the base portions 126, 226, and 326 (see FIGS. 1B, 2B, and 3B), and the tip portions 124, 224, and 324 are inserted into the skin and then dissolved by a chemical material. Accordingly, the substrates 110, 210, and 310 may be chemically separated from the tip portions 124, 224, and 324 (see FIGS. 1A, 2A, and 3A), the tip portions 124, 224, and 324 may be chemically separated from the base portion 126, 226, and 326 (see FIGS. 1B, 2B, and 3B), or the substrates 110, 210, and 310 may be chemically separated from the base portions 126, 226, and 326 (see FIGS. 1C, 2C, and 3C).

The chemical material may include at least one selected from among water, an aqueous solution, and an injectable ester, the aqueous solution may include at least one selected from among anhydrous or aqueous lower alcohol having one to four carbon atoms, acetone, ethyl acetate, chloroform, 1,3-butylene glycol, hexane, diethyl ether, and butyl acetate, and the injectable ester may include at least one selected from among a water-insoluble agent, propylene glycol, polyethylene glycol, an oily component, and ethyl oleate. Here, the oil that is mainly usable as an oily component may include at least one selected from among a vegetable oil, a mineral oil, a silicone oil, and a synthetic oil.

In this case, solubility of the separation portions 122, 222, and 322 with respect to the chemical material may be greater than that of the tip portions 124, 224, and 324 and/or the base portions 126, 226, and 326.

In this way, the separation portions 122, 222, and 322 may be formed of a material having high solubility so that the separation portions 122, 222, and 322 are easily dissolved by the chemical material added as a solvent and the tip portions 124, 224, and 324 are separated. For example, the separation portions 122, 222, and 322 may include at least one selected from among biodegradable polymers, such as hyaluronic acid or carboxymethylcellulose, and sugars such as glucose or sucrose.

In addition, when the separation portions 122, 222, and 322 are not completely dissolved by the chemical material and a portion of each of the separation portions 122, 222, and 322 remains on one sides of the tip portions 124, 224, and 324 (see FIGS. 1A, 1C, 2A, 2C, 3A, and 3C) or remains on one sides of the base portions 126, 216, and 326 (see FIGS. 1B, 2B, and 3B), the separation portions 122, 222, and 322 may include a biodegradable material so that the portion may be dissolved by the skin.

In this case, a dissolution time of may be less than five minutes for the separation portions 122, 222, and 322 by the chemical material to separate the substrates 110, 210, and 310 from the tip portions 124, 224, and 324 (see FIGS. 1A, 2A, and 3A), separate the base portions 126, 226, and 326 from the tip portions 124, 224, and 324 (see FIGS. 1B, 2B, and 3B), or separate the substrate 110, 210, and 310 from the base portion 126, 226, and 326 (see FIGS. 1C, 2C, and 3C). That is, the separation portions 122, 222, and 322 may be dissolved by the chemical material so that the substrates 110, 210, and 310 are completely separated from the tip portions 124, 224, and 324 (refer to FIGS. 1A, 2A, and 3A), the base portion 126 are completely separated from the tip portions 124, 224, and 324 (see FIGS. 1B, 2B, and 3B), or the substrates 110, 210, and 310 are completely separated from the base portions 126, 226, and 326 (see FIGS. 1C, 2C, and 3C) within a certain period of time. Here, when the time required for the separation portions 122, 222, and 322 to be dissolved is five minutes or more, side effects such as skin irritation and inflammation may occur depending on a user, like the micro-patch of the related art.

In this case, materials forming the separation portions 122, 222, and 322 and kind and concentration of the chemical material may be selected so that the separation portions 122, 222, and 322 are dissolved to sufficiently separate the substrates 110, 210, and 310 from the tip portions 124, 224, and 324 (FIGS. 1A, 2A, and 3A), separate the base portion 126 from the tip portion 124, 224, and 324 (see FIGS. 1B, 2B, and 3B), or separate the substrates 110, 210, and 310 from the base portions 126, 226, and 326 (see FIGS. 1C, 2C, and 3C), within a certain period of time.

In addition, the separation portions 122, 222, and 322 may be dissolved by the chemical material by 70% or more per unit volume within three minutes. Here, even when the separation portions 122, 222, and 322 are completely dissolved, the separation portions 122, 222, and 322 may separate the substrates 110, 210, and 310 from the tip portions 124, 224, and 324 (see FIGS. 1A, 2A, and 3A), separate the base portions 126, 226, and 326 from the tip portion (124, 224, 324) (see FIGS. 1B, 2B, and 3B), or separate the substrates 110, 210, and 310 from the base portions 126, 226, and 326 (see FIGS. 1C, 2C, and 3C) by a small external force. That is, when the separation portions 122, 222, and 322 are dissolved by 70% or more per unit volume, coupling forces with the substrates 110, 210, and 310 and the tip portions 124, 224, and 324 are weakened (see FIGS. 1A, 2A, and 3A), coupling forces with the base portion 126, 226, and 326 and the tip portions 124, 224, and 324 are weakened (see FIGS. 1B, 2B, and 3B), or coupling forces with the substrates 110, 210, and 310 and the base portions 126, 226, and 326 are weakened (see FIGS. 1C, 2C, and 3C), and thus, the separation portions 122, 222, and 322 may be broken even by a small external force.

Here, when a time required to dissolve a certain volume of each of the separation portions 122, 222, and 322 is three minutes or more, like the micro-patch of the related art, side effects such as the skin irritation and inflammation may occur depending on the user.

In addition, in a case where the separation portions 122, 222, and 322 are dissolved by less than 70% per unit volume, when the external force for breaking the separation portion 122, 222, and 322 is greater than the coupling forces between the separation portions 122, 222, and 322 and the tip portions 124, 224, and 324 (FIGS. 1A, 2A, and 3A), greater than the coupling forces between the separation portions 122, 222, and 322 and the tip portions 124, 224, and 324 or the base portions 126, 226, and 326 (see FIGS. 1B, 2B, and 3B), greater than the bolding forces between the separation portions 122, 222, and 322 and the substrates 110, 210, and 310 or the base portions 126, 226, and 326 (see FIGS. 1C, 2C, and 3C), or greater than coupling forces between the tip portions 124, 224, 324 and the skin, the tip portions 124, 224, 324 may be separated from the skin.

In this case, the materials forming the separation portions 122, 222, and 322 and the kind and concentration of the chemical material may be selected so that the separation portions 122, 222, and 322 are dissolved by a certain amount or more within a certain period of time and broken even by a small external force.

The tip portions 124, 224, and 324 may include the drug as described above. In addition, the tip portions 124, 224, and 324 may be a material having high solubility by body fluid so that the loaded drug is easily delivered to the skin after the tip portions 124, 224, and 324 are inserted. That is, the tip portions 124, 224, and 324 may include a biocompatible material or a biodegradable material.

The base portions 126, 226, and 326 may be formed on the substrates 110, 210, and 310 or the separation portions 122, 222, and 322 in a cylindrical shape having a circular cross section. Here, the base portions 126, 226, and 326 may be formed so that the cross-sectional areas decrease in a direction from the substrates 110, 210, and 310 toward the separation portions 122, 222, and 322 but are not limited thereto. In this case, the base portions 126, 226, and 326 may not be dissolved by the chemical material or may be formed of a material having solubility lower than the separation portions 122, 222, and 322.

The base portions 126, 226, and 326 have a function of providing a supporting force by which the tip portions 124, 224, and 324 is completely insertable into skin 1. Therefore, the base portions 126, 226, and 326 may not contain the drug included in the tip portions 124, 224, and 324. Accordingly, the tip portions 124, 224, and 324 are completely inserted into the skin 1, and thus, the amount of the drug administered to the skin 1 may be maintained and managed to be constant.

Alternatively, when the base portions 126, 226, and 326 are formed on the tip portions 124, 224, and 324, the base portions 126, 226, and 326 may be formed integrally with the tip portions 124, 224, and 324 or may include the drug contained in the tip portions 124, 224, and 324. Accordingly, the amount of the drug administered to the skin 1 may increase as compared to a case where the drug is included only in the tip portions 124, 224, and 324.

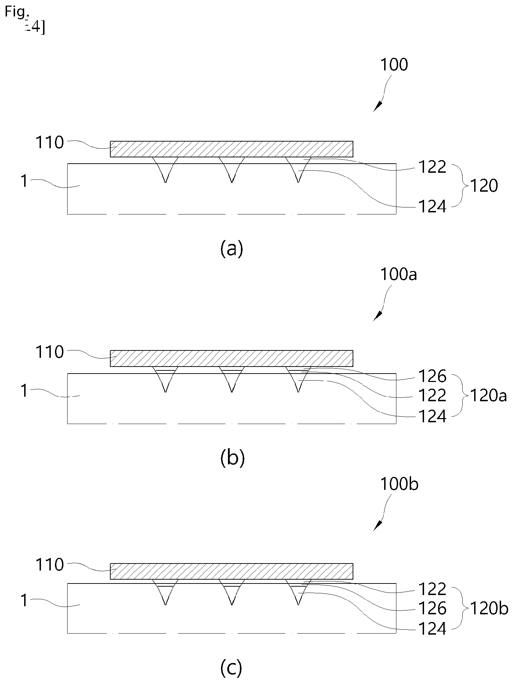

Hereinafter, a process in which the microstructure is inserted into the skin 1 by chemically separating the separation portions 122, 222, and 322 will be described with reference to FIGS. 4 to 8.

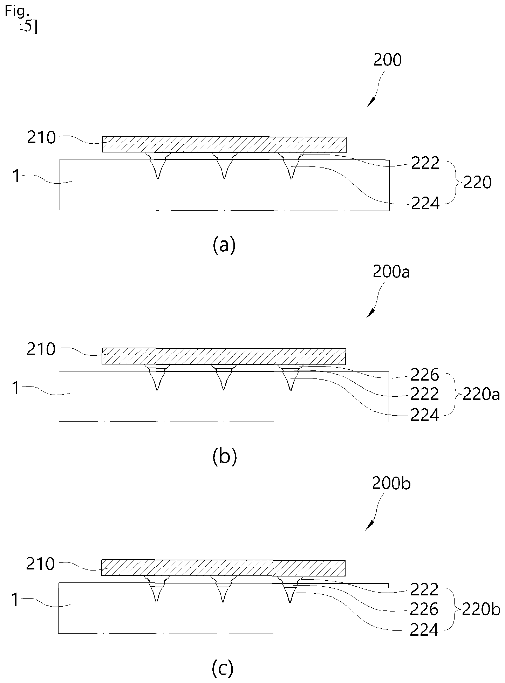

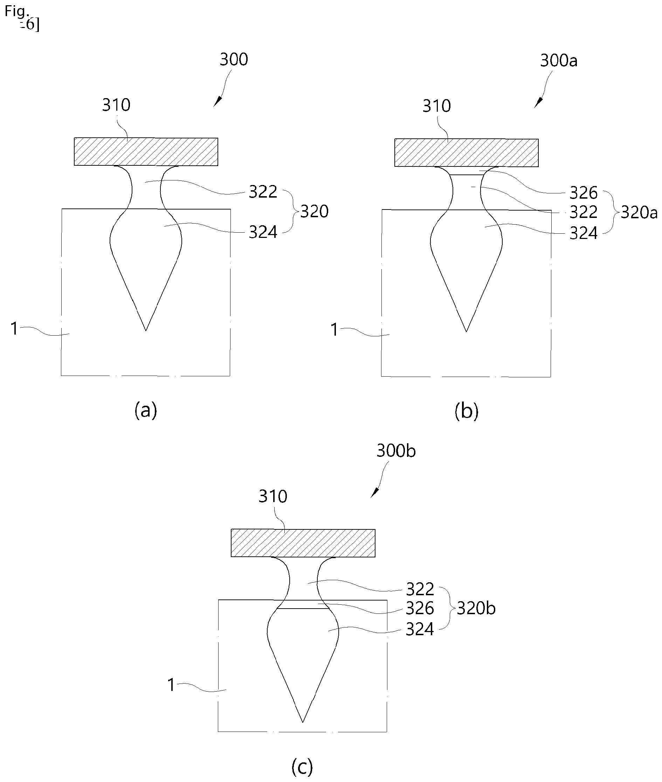

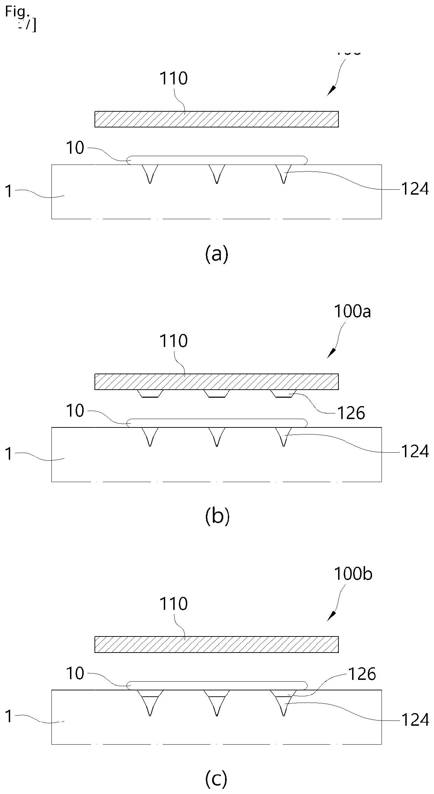

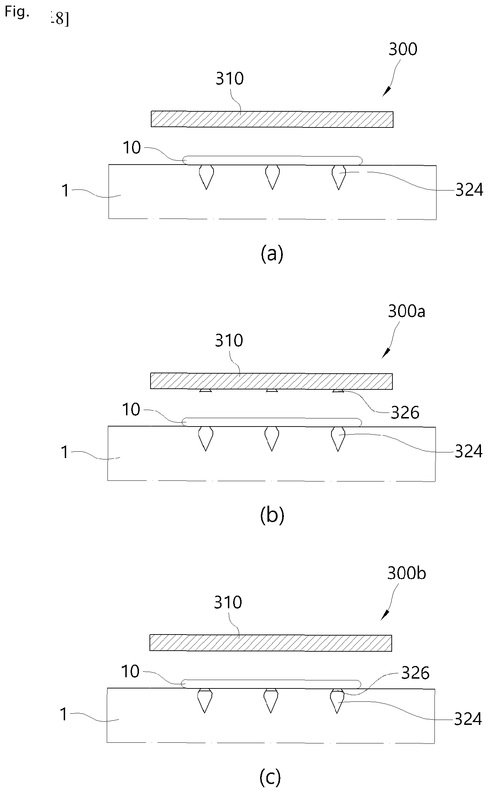

FIG. 4 is a cross-sectional view illustrating a state in which the microstructure of FIG. 1 is inserted into the skin, FIG. 5 is a cross-sectional view illustrating a state in which the microstructure of FIG. 2 is inserted into the skin, FIG. 6 is a cross-sectional view illustrating a state in which the microstructure of FIG. 3 is inserted into skin, FIG. 7 is a cross-sectional view for describing a process of chemically separating the separation portion in FIG. 4, and FIG. 8 is a cross-sectional view for describing a process of chemically separating the separation portion in FIG. 6.

Referring to FIGS. 4 to 6, the micro-patch is pressed against the skin 1 so that the tip portions 124, 224, and 324 are inserted into the skin 1. In this case, the substrates 110, 210, and 310 are pressed with a uniform force so that the tip portions 124, 224, and 324 are sufficiently inserted into the skin 1, and at the same time, are uniformly inserted with respect to the entireties of the substrates 110, 210, and 310.

In this case, as illustrated in FIGS. 4A, 5A, and 6A, when the base portions 126, 226, and 326 are not present, only the tip portions 124, 224 and 324 may be inserted into the skin 1.

Similarly, as illustrated in FIGS. 4B, 5B, and 6B, when the base portions 126, 226, and 326 are formed on the substrates 110, 210, and 310, only the tip portions 124, 224, and 324 may be inserted into the skin 1. In this case, the base portions 126, 226, and 326 may provide a supporting force so that the tip portions 124, 224, and 324 may be completely inserted into the skin 1.

In addition, as illustrated in FIGS. 4C, 5C, and 6C, when the base portions 126, 226, and 326 are formed on the separation portions 122, 222, and 322, the base portions 126, 226, and 326 together with the tip portions 124, 224, and 324 may be inserted into the skin 1. In this case, the base portions 126, 226, and 326 may include a biodegradable material.

However, the present invention is not limited thereto, and the base portions 126, 226, and 326 may not be inserted into the skin 1 and only the tip portions 124, 224, and 324 may be inserted into the skin 1.

Referring to FIGS. 7 and 8, as described above, the chemical material 10 is introduced between the substrates 110 and 310 and the skin 1, and the separation portions 122 and 322 are dissolved within a few minutes by the chemical material 10. Here, in the microstructures 220, 220a, and 220b according to the second embodiment, states of the microstructures 220, 220a, and 220b after the separation portion 222 is removed by the chemical material 10 are the same as those of the microstructures 120, 120a, and 120b according to the first embodiment, and thus, the drawings and description are omitted here.

In this case, as illustrated in FIGS. 7A and 8A, when the base portions 126 and 326 are not present, the separation portions 122 and 322 are dissolved and there may be no residue on the substrates 110 and 310.

Similarly, as illustrated in FIGS. 7C and 8C, when the base portions 126 and 326 are formed on the separation portions 122 and 322, the separation portions 122 and 322 are dissolved and there may be no residue on the substrates 110 and 310.

In addition, as illustrated in FIGS. 7B and 8B, when the base portions 126 and 326 are formed on the substrates 110 and 310, the separation portions 122 and 322 are dissolved and the base portions 126 and 326 may remain on the substrates 110 and 310 as residue.

Here, the chemical material 10 may be introduced between the substrate 110 and the skin 1 in a state in which the microstructure is inserted into the skin. As another example, the chemical material 10 may be first applied onto the skin 1 before the tip portions 124 and 324 are inserted into the skin 1.

In this case, the dissolution rate and dissolution amount of the separation portions 122 and 322 are determined depending on the types and concentrations of the material forming the separation portions 122 and 322 and the chemical material 10, and according to the determined conditions, the separation portions 122 and 322 may be completely dissolved within a certain period of time or may be dissolved to an extent that the separation portions 122 and 322 may be separated by a small external force.

In this way, the separation portions 122 and 322 are completely dissolved by the chemical material 10 or dissolved in a predetermined amount or more, and thus, the tip portions 124 and 324 are separated (see FIGS. 7A and 8A), the base portions 126 and 326 formed on the substrates 110 and 310 and the tip portions 124 and 324 are separated (see FIGS. 7B and 8B), or the base portions 126 and 326 are separated (see FIGS. 7C and 8C). Accordingly, the substrates 110 and 310 may be removed in a state in which the tip portions 124 and 324 are inserted into the skin 1.

In this case, when the separation portions 122 and 322 are not completely dissolved by the chemical material 10 and only a certain amount thereof is dissolved, the substrates 110 and 310 are not completely separated from the tip portions 124 and 324 (see FIGS. 7A and 8A), the base portions 126 and 326 are not completely separated from the tip portions 124 and 326 (see FIGS. 7B and 8B), or the substrates 110 and 310 are not completely separated from the base portions 126 and 326 (FIGS. 7C and 8C). However, the separation portions 122 and 322 may be broken by applying a small external force to the substrates 110 and 310.

Accordingly, since the tip portions 124 and 324 are separatable from the substrates 110 and 310 within a short time, an attachment time of the micro-patch can be shortened. Therefore, it is possible to reduce or prevent possible side effects such as skin marks and inflammation caused by a long-term application of the conventional micro-patch, and thus, stability and convenience of use can be improved.

In addition, since it is possible to prevent a decrease in a skin absorption rate of the drug due to sweat, heat generation, inability of fixing with respect to the skin, or the like caused when the conventional micro-patch is used for a long time, the same drug absorption rate and efficacy can be provided for each subject to which the patch is to be applied.

In addition, since the attachment time of the micro-patch is short, the drug can be delivered regardless of skin conditions, an application site, and an environment of the subject to which the patch is to be applied, and thus, it is possible to improve the supply of substances.

Furthermore, despite the short attachment time of the micro-patch, since there is no need to use a separate shooting device for injecting the drug into the skin, it is possible to maximize advantages of no pain and no irritation.

Accordingly, the tip portions 124 and 324 containing the drug are dissolved in a state of being sufficiently inserted into the skin 1, and thus, the drug can be absorbed into the skin 1.

As a result, since the tip portions 124 and 324 containing the drug are not dissolved at outside of the skin 1, there is no residue on the substrates 110 and 310, and all tip portions 124 and 324 are absorbed into the skin, quantitative delivery of the drug can be realized. Therefore, the microstructure can ensure the quantitative administration of the drug, and thus, can be applied in a field of vaccination, such as a vaccine, which is more sensitive to dosage.

In addition, the drug in the tip portions 124 and 324 does not remain on the substrates 110 and 310. Accordingly, the micro-patch of the present invention is hygienic compared to the conventional micro-patch, and thus, it is possible to minimize incidences of biohazard to the subject to which the patch is to be applied, biohazardous waste, and secondary damages thereof.

Meanwhile, the present invention can provide the microstructure that is easily separated by a small physical force.

Referring again to FIGS. 1 to 3, the separation portions 122, 222, and 322 may physically separate the substrates 110, 210, and 310 from the tip portions 124, 224, and 324 (FIGS. 1A, 2A, and 3A), separate the base portion 126, 226, and 326 from the tip portion 124, 224, and 324 (see FIGS. 1B, 2B, and 3B), or separate the substrate 110, 210, and 310 from the tip portion 124, 224, and 324 (see FIGS. 1A, 2C, and 3C) by an external force after the tip portions 124, 224, and 324 are inserted into the skin. Here, the external force applied to the separation portions 122, 222, and 322 may be applied to the separation portions 122, 222, and 322 in a horizontal direction or a vertical direction.

In this case, the case where the external force is applied to the separation portions 122, 222, and 322 in the horizontal direction may be a case where the substrate 110 is pushed in a direction parallel to the skin. In addition, the case where the external force is applied to the separation portions 122, 222, and 322 in the vertical direction may be a case where the substrate 110 is pulled in a direction opposite to the skin.

Preferably, in the separation portions 222 and 322, central portions thereof have cross sections smaller than cross sections of interfaces with the base portions 226 and 326 or the substrates 210 and 310 and cross sections of interfaces with the tip portions 224 and 324. Accordingly, the separation portions 222 and 322 may be easily broken even with a small physical force. For example, the separation portions 222 and 322 may have a width smaller than the base portions 226 and 326 and the tip portions 224 and 324 and thus may have a concave shape in a cross-sectional view.

In this case, the separation portions 222 and 322 may be broken by a force smaller than coupling forces between the substrates 210 and 310 and the tip portions 224 and 324 (see FIGS. 2A and 3A), may be broken by a force smaller than coupling forces between the base portions 226 and 326 and the tip portions 224 and 324 (see FIGS. 2B and 3B), or may be broken by a force smaller than coupling forces between the substrates 210 and 310 and the tip portions 224 and 324 (see FIGS. 2C and 3C).

That is, when the force smaller than the coupling forces between the substrates 210 and 310 and the tip portions 224 and 324 is applied to the separation portions 222 and 322, the separation portions 222 and 322 are not separated from the substrates 210 and 310 and the tip portions 224 and 324, and both sides of each of the separations portions 222 and 322 are firmly fixed to the substrates 210 and 310 and the tip portions 224 and 324. Accordingly, the separation portions 222 and 322 may be easily broken by the applied external force (see FIG. 2A and FIG. 3A).

Similarly, when the force smaller than the coupling forces between the base portions 226 and 326 and the tip portions 224 and 324 is applied to the separation portions 222 and 322, the separation portions 222 and 322 are not separated from the base portions 226 and 326 and the tip portions 224 and 324, and both sides of each of the separations portions 222 and 322 are firmly fixed between the base portions 226 and 326 and the tip portions 224 and 324. Accordingly, the separation portions 222 and 322 may be easily broken by the applied external force (see FIG. 2B and FIG. 3B).

Similarly, when the force smaller than the coupling forces between the substrates 210 and 310 and the base portions 226 and 326 is applied to the separation portions 222 and 322, the separation portions 222 and 322 are not separated from the substrates 210 and 310 and the base portions 226 and 326, and both sides of each of the separations portions 222 and 322 are firmly fixed between the substrates 210 and 310 and the base portions 226 and 326. Accordingly, the separation portions 222 and 322 may be easily broken by the applied external force (see FIG. 2C and FIG. 3C).

In addition, the separation portions 222 and 322 may be broken by a force smaller than a force by which the tip portions 224 and 324 are separated from the skin. That is, when the force smaller than the force by which the tip portions 224 and 324 are separated from the skin is applied to the separation portions 222 and 322, in a state in which the tip portions 224 and 324 is not be separated from the skin, both sides of each of the separation portions 222 and 322 may be firmly fixed between the substrates 210 and 310 and the tip portions 224 and 324 (see FIGS. 2A and 3A), may be firmly fixed between the base portions 226 and 326 and the tip portions 224 and 324 (see FIGS. 2B and 3B), or may be firmly fixed between the substrates 210 and 310 and the base portions 226 and 326 (see FIGS. 2C and 3C). Accordingly, the separation portions 222 and 322 may be easily broken by the applied external force.

For example, the separation portions 222 and 322 may have a strength of 0.01 to 100 N. Here, when the strength is less than 0.01 N, the separation portions 222 and 322 may be broken before the tip portions 224 and 324 are sufficiently inserted into the skin while the tip portions 224 and 324 are inserted into the skin. In addition, when the strength is 100 N or more, the strength is greater than the coupling force between the tip portions 224 and 324 and the skin. Accordingly, the tip portions 224 and 324 may be separated from the skin before the separation portions 222 and 322 are broken.

In this case, in the case where the base portions 226 and 326 are formed, the base portions 226 and 326 may be formed of a material having high strength to transmit a force by which the separation portions 222 and 322 can be broken while not separated from the separation portions 222 and 322 when the tip portions 224 and 324 are inserted into the skin and then a physical force is applied to the base portions 226 and 326.

Meanwhile, even when the external force is insufficient and the separation portions 222 and 322 are not completely broken, the separation portions 222 and 322 may be separated from the interface with the substrates 210 and 310, the base portions 226 and 326, or the tip portions 224 and 324 by the coupling forces between the separation portions 222 and 322 and the substrates 210 and 310, the coupling forces between the separation portions 222 and 322 and the base portions 226 and 326, or the coupling forces between the separation portions 222 and 322 and the tip portions 224 and 324.

For example, as illustrated in FIGS. 2B and 3B, when the base portions 226 and 326 are formed on the substrates 210 and 310, the coupling forces between the separation portions 222 and 322 and the base portions 226 and 326 may be smaller than the coupling forces between the base portions 226 and 326 and the substrate 110. In this case, even when the separation portions 222 and 322 are not broken by the applied external force, the separation portions 222 and 322 may be separated from the base portions 226 and 336 at the interfaces with the base portions 226 and 326 or in the vicinities of the interfaces.

In this case, the separation portions 222 and 322 may not be completely broken to be removed but may partially remain on the tip portions 224 and 324. Therefore, the separation portions 222 and 322 may include a biodegradable material that can be dissolved by the skin.

In addition, the coupling forces between the separation portions 222 and 322 and the tip portions 224 and 324 may be smaller than the coupling forces between the separation portions 222 and 322 and the base portions 226 and 326. In this case, even when the separation portions 222 and 322 are not broken by the applied external force, the separation portions 222 and 322 may be separated from the tip portions 224 and 324 at the interfaces with the tip portions 224 and 324 or in the vicinities of the interfaces.

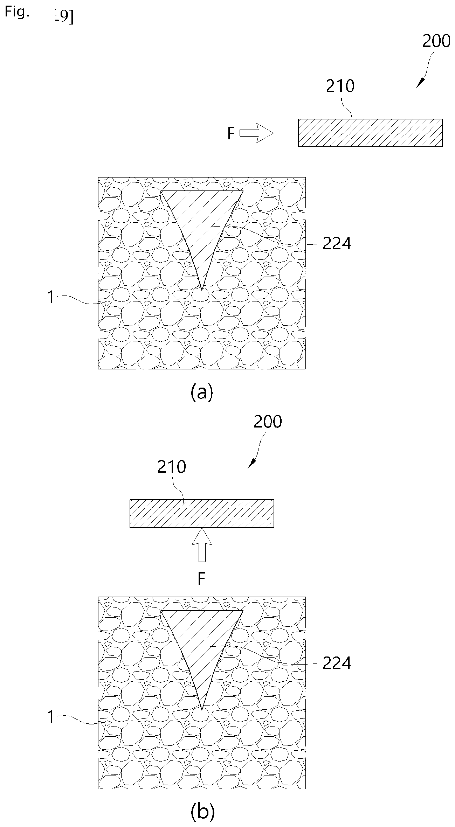

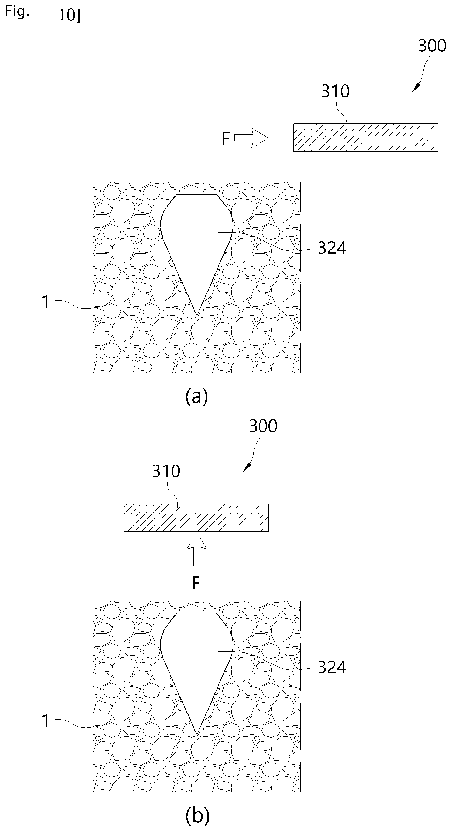

Hereinafter, a process in which the microstructure is inserted into the skin 1 by physically separating the separation portions 222 and 322 will be described with reference to FIGS. 9 and 10.

FIG. 9 is a cross-sectional view illustrating a process of physically separating the separation portion in FIG. 4, and FIG. 10 is a cross-sectional view illustrating a process of physically separating the separation portion in FIG. 6.

Referring again to FIGS. 4 and 6, the micro-patch is pressed against the skin 1 so that the tip portions 224 and 324 are inserted into the skin 1. In this case, the substrates 210 and 310 are pressed with a uniform force so that the tip portions 224 and 324 are sufficiently inserted into the skin 1 and at the same time, are uniformly inserted with respect to the entireties of the substrates 210 and 310.

Referring to FIGS. 9 and 10, the separation portions 222 and 322 are broken by a force F caused by physically applying a force F to the substrates 210 and 310 in the horizontal or vertical direction. In this case, applying the physical force in the horizontal direction may include applying a force in the vertical direction together with the horizontal direction such as repeatedly applying a force in the horizonal direction such as rubbing with a finger after applying the patch, and turning the patch clockwise or counterclockwise while applying the patch vertically with a finger. In addition, applying the physical force in the vertical direction may include separating the patch from the skin, pressing the patch to the skin to apply a pressure, and applying a force in the horizontal direction together with the vertical direction as described above. Here, in the microstructures 120, 120a, and 120b according to the first embodiment, the states of the microstructures 120, 120a, and 120b after the separation portion 122 is broken are the same as those of the microstructures 220, 220a, and 220b according to the second embodiment, and thus, the drawings and description are omitted here.

In this case, a physical force F applied from the outside to break the separation portions 222 and 322 is determined according to the shape, material, size, or the like of the microstructure, and according to the determined conditions, the separation portions 222 and 322 may be broken, separated from the interfaces with the substrates 210 and 310 or the tip portions 224 and 324, separated from the interfaces with the base portions 226 and 326 or the tip portions 224 and 324, or separated from the interfaces with the substrates 210 and 310 or the base portions 226 and 326.

In this way, the separation portions 222 and 322 are broken, separated from the substrates 210 and 310, or separated from the base portions 226 and 326 by the externally applied force F. Accordingly, the substrates 210 and 310 are separated from the tip portions 224 and 324, and thus, the substrates 210 and 310 may be removed in a state in which the tip portions 224 and 324 are inserted into the skin 1.

In this case, when the separation portions 222 and 322 are separated from the substrates 210 and 310 or separated from the base portions 226 and 326 without being broken by the externally applied force F, the separation portions 222 and 322 are at least partially inserted into the skin 1 together with the tip portions 224 and 324 but may be dissolved by the skin 1 because the separation portions 224 and 324 include a biodegradable material.

Alternatively, the separation portions 222 and 322 remaining on one side of each of the tip portions 224 and 324 or one side of each of the base portions 226 and 326 may be dissolved by a chemical material by applying the chemical material to the skin.

Meanwhile, the microstructure 320a according to the third embodiment may store a large amount of drugs as compared to the conventional microstructure having the same shape as that of the microstructure according to the first and second embodiments. This is according to shape characteristics of the tip portion of the microstructure 320a according to the present embodiment. The drug that may be contained in an upper 60% of the height range corresponding to the tip portion in the height direction of the conventional microstructure is only about 31%. However, the microstructure 320a according to the present embodiment may contain about 60% of the drug in the tip portion, which is greater than that of the conventional microstructure. In this case, a ratio of the drug that the microstructure 320a may contain may vary depending on the shapes of the tip portion and the separation portion. As an example, the microstructure 320a may have a volume of 60 to 90% of the total volume in the height range of the upper 60% in the height direction but is not limited thereto.

In addition, the microstructure 320a is formed so that the tip portion 324 has a cross section wider than that of the separation portion 322 and is formed in a substantially spherical or oval shape to store a large amount of drugs therein. Accordingly, in a state in which the tip portion 324 is completely inserted into the skin, the separation portion 322 is cut in the transverse direction so that the tip portion 324 is positioned inside the skin, and thus, a large amount of drug may be injected into the skin.

In addition, in the case of the conventional microstructure, there is a problem in that the microstructure including the substrate should be in contact with the skin for a long time until the drug penetrates the skin in a state in which the drug is located inside the microstructure. However, in the microstructure 320a, the separation portion 322 is quickly cut in the state in which the tip portion 324 is inserted into the skin to position the tip portion 324 inside the skin. Accordingly, it is possible to reduce a time required to bring the substrate 310 in contact with the skin to insert the tip portion 324 into the skin.

In addition, the microstructure 320a is not easily removed from the skin in a state in which a portion having the widest cross section of the tip portion 324 is inserted into the skin. The microstructure 320a lifts and supports the skin without being removed from the skin when an attempt is made to remove the microstructure 320a from the skin with a weak force. This is because after the tip portion 324 of the microstructure 320a is inserted into the skin, the tip portion 324 may be fixed inside the skin due to elasticity of the skin.

The tip portion 324 of the microstructure 320a inserted into the skin is fixed into the skin, and the larger the widest cross-section of the tip portion 324 is compared with the cross-section of the separation portion 322, the more difficult it is to remove the separation portion 322 from the skin. That is, the larger and wider the tip portion 324 is, the greater the magnitude of the force for removing the tip portion 324 from the inside of the skin. In comparison, in the conventional microstructure, the cross section is widened in a direction toward the substrate in the state in which the tip of the microstructure is inserted inside the skin, and thus, the microstructure may be easily removed or separated from the skin even by a small force.

In comparison, the microstructure 320a is not easily removed from the skin in the state in which tip portion 324 is inserted into the skin. Accordingly, when the separation portion 322 is broken in the transverse direction in the state in which the tip portion 324 is inserted into the skin and the state in which the tip portion 324 being inserted into the skin is maintained, the drug inside the tip portion 324 can penetrate into the skin, and thus, it is possible to simply administer the drug in the microstructure into the skin.

In addition, in the microstructure 320a, the patch is separated by a predetermined force immediately after the substrate, that is, the patch to which the microstructure is coupled, comes into contact with the skin or after a short period of time elapses. Accordingly, it is convenient when the user administers the drug using the microstructure. However, the conventional microstructure has disadvantages in that the patch should be in contact with the skin until the drug contained in the microstructure is continuously injected into the skin and no further injection is made.

In addition, in the patch having the conventional microstructure, in order to inject the drug in the microstructure into the skin, an adhesive material is applied or attached to the patch or substrate to maintain the state attached to the skin when used. Accordingly, the user may feel a foreign body sensation during use or pain when the patch or substrate is removed from the skin. However, in the substrate or patch having the microstructure 320a, after the microstructure is inserted in the skin, the substrate or patch is separated from the microstructure with a predetermined force. Accordingly, a separate adhesive material or adhesive layer is not required, and thus, it is easy to use.

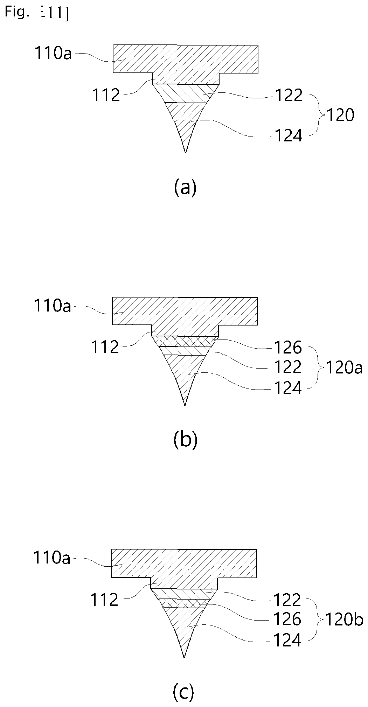

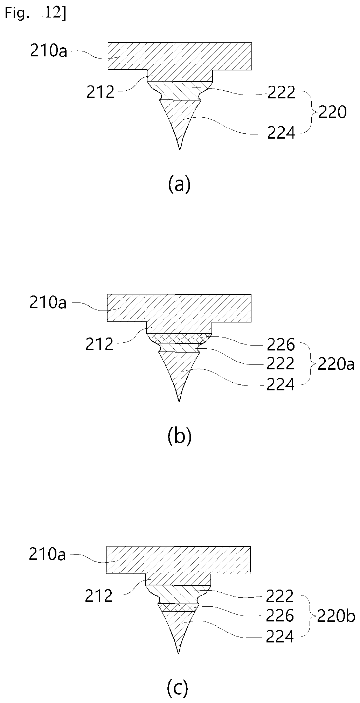

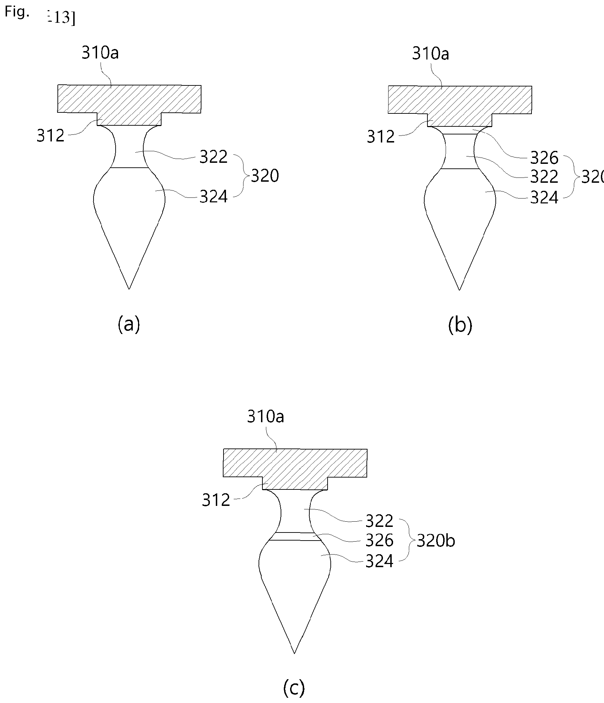

Meanwhile, the microstructures 120, 220, and 320 may be formed on the substrate on which the protrusion is formed. FIG. 11 is a cross-sectional view of a modification example of the microstructure according to the first embodiment of the present invention, FIG. 12 is a cross-sectional view of a modification example of the microstructure according to the second embodiment of the present invention, and FIG. 13 is a cross-sectional view of a modification example of the microstructure according to the third embodiment of the present invention.

Referring to FIG. 11, the microstructures 120, 120a, and 120b having a substantially conical shape may be formed on a substrate 110a having a protrusion 112. In this case, in the substrate 110a, the protrusion 112 may be formed on a base of a portion (the separation portion 122 of FIGS. 11A and 11C or the base portion 126 of FIG. 11B) corresponding to the microstructures 120, 120a, and 120b. Here, the protrusion 112 may be formed to have a predetermined thickness from one surface of the substrate 110a. Accordingly, the microstructures 120, 120a, and 120b may be more easily inserted into the skin because a supporting force by the substrate 110a increases.

In addition, in the protrusion 112, a surface on which the microstructures 120, 120a, and 120b are formed may have an arbitrary shape. For example, in the protrusion 112, the surface on which the microstructures 120, 120a, and 120b are formed may have a horizontal straight line, a diagonal line inclined to one or both sides, and a convex or concave curve in the center in a cross-sectional view. Accordingly, the microstructures 120, 120a, and 120b may be easily separated from the substrate 110a because the coupling force with the protrusion 112 is reduced.

Referring to FIG. 12, the microstructures 220, 220a and 220b having a substantially conical shape and a concave shape at an approximately central portion may be formed on a substrate 210a having a protrusion 212. In this case, in the substrate 210a, the protrusion 212 may be formed on a portion (the separation portion 222 of FIGS. 12A and 12C or the base portion 226 of FIG. 12B) corresponding to the microstructures 220, 220a, and 220b. Here, the protrusion 212 may be formed to have a predetermined thickness from one surface of the substrate 210a. Accordingly, the microstructures 220, 220a, and 220b may be more easily inserted into the skin because a supporting force by the substrate 210a increases.

In addition, in the protrusion 212, a surface on which the microstructures 220, 220a, 220b are formed may have an arbitrary shape. For example, in the protrusion 212, the surface on which the microstructures 120, 120a, and 120b are formed may have a horizontal straight line, a diagonal line inclined to one or both sides, and a convex or concave curve in the center in a cross-sectional view. Accordingly, the microstructures 220, 220a, and 220b may be easily separated from the substrate 210a because the coupling force with the protrusion 212 is reduced.

Referring to FIG. 13, the microstructures 320, 320a, and 320b which are substantially candle-shaped may be formed on a substrate 310a having a protrusion 312. In this case, in the substrate 310a, a protrusion 312 may be formed on a portion (the separation portion 322 of FIGS. 13A and 13C or the base portion 326 of FIG. 13B) corresponding to the microstructures 320, 320a, and 320b. Here, the protrusion 312 may be formed to have a predetermined thickness from one surface of the substrate 310a. Accordingly, the microstructures 320, 320a, and 320b may be more easily inserted into the skin because a supporting force by the substrate 310a increases.

In addition, in the protrusion 312, a surface on which the microstructures 320, 320a, and 320b are formed may have an arbitrary shape. For example, in the protrusion 312, the surface on which the microstructures 320, 320a, and 320b are formed may have a horizontal straight line, a diagonal line inclined to one or both sides, and a convex or concave curve in the center in a cross-sectional view. Accordingly, the microstructures 320, 320a, and 320b may be easily separated from the substrate 310a because a coupling force with the protrusion 112 is reduced.

As described above, according to the present invention, it is possible to shorten the attachment time of the micro-patch, reduce side effects such as skin irritation and inflammation, and improve stability and convenience of use.

In addition, according to the present invention, it is possible to prevent a decrease in a skin absorption rate of the drug due to sweat, heat generation, inability of fixing with respect to the skin, or the like. Therefore, it is possible to provide a uniform drug absorption rate and efficiency, the drug can be delivered regardless of skin conditions, an application site, and an environment of the subject to which the patch is to be applied, and it is possible to improve the supply of substances.

In addition, according to the present invention, there is no need to use a separate shooting device for injecting the drug into the skin, and thus, it is possible to maximize advantages of no pain and no irritation.

In addition, according to the present invention, the tip portion containing the drug is completely inserted into the skin. Therefore, the tip portion is not dissolved at outside of the skin, there is no residue of the micro-patch, all of the tip portion is absorbed into the skin, and thus, quantitative delivery of the drug can be realized.

In addition, the drug in the tip portion does not remain on the micro-patch. Accordingly, the micro-patch of the present invention is hygienic compared to the conventional micro-patch, and thus, it is possible to minimize incidences of biohazard of the subject to which the patch is to be applied, biohazardous waste, and secondary damages thereof.

Heretofore, the embodiments of the present invention are described. However, a spirit of the present invention is not limited to the embodiments presented in the present specification, those skilled in the art who understand the spirit of the present invention can easily implement other embodiments by adding, changing, deleting, or adding components within a scope of the same idea, and other embodiment, and other embodiments also fall within the scope of the present invention.

## Claims

1. A microstructure comprising:
a tip portion including a drug; and
a separation portion which is formed between a substrate and the tip portion and, after the tip portion is inserted into a skin, separates the substrate from the tip portion physically by an external force or chemically by a chemical material.

2. The microstructure of claim 1, further comprising a base portion formed on one side of the substrate or the tip portion,
wherein the separation portion is formed between the base portion and the tip portion or between the substrate and the base portion, and after the tip portion is inserted into the skin, separates the base portion from the tip portion or separates the substrate from the base portion.

3. The microstructure of claim 1, wherein the separation portion includes at least one selected from among a biodegradable polymer and sugar.

4. The microstructure of claim 1, wherein a dissolution time of the separation portion is less than five minutes by the chemical material, or the separation portion is dissolved by the chemical material by 70% or more per unit volume within three minutes.

5. The microstructure of claim 1, wherein the chemical material is introduced between the substrate and the skin or is applied on the skin before the tip portion is inserted into the skin.

6. The microstructure of claim 1, wherein the chemical material includes at least one selected from among water, an aqueous solution, and an injectable ester,
the aqueous solution includes at least one selected from among anhydrous or aqueous lower alcohol having carbon atoms of one to four, acetone, ethyl acetate, chloroform, 1,3-butylene glycol, hexane, diethyl ether, and butyl acetate,
the injectable ester includes at least one selected from among a water-insoluble agent, propylene glycol, polyethylene glycol, an oily component, and ethyl oleate, and
an oil useable as the oily component includes at least one selected from among a vegetable oil, a mineral oil, a silicone oil, and a synthetic oil.

7. The microstructure of claim 1, wherein in the separation portion, a cross section of a central portion is smaller than cross sections of both sides.

8. The microstructure of claim 1, wherein the separation portion is broken by a force smaller than a coupling force with the tip portion or the substrate.

9. The microstructure of claim 1, wherein the separation portion is broken by a force smaller than a force by which the tip portion is separated from the skin.

10. The microstructure of claim 1, wherein the external force is applied to the separation portion in a horizontal direction or a vertical direction.

11. The microstructure of claim 1 or 2, wherein the separation portion or the base portion contains a biodegradable material.

12. The microstructure of claim 1, wherein the tip portion has a candle shape of which a vertical cross section decreases in a direction toward a tip or has a continuous curvature.

13. The microstructure of claim 1, wherein the substrate includes a protrusion having a predetermined thickness at a portion of the substrate corresponding to the separation portion and is formed of a horizontal straight line, an inclined oblique line, or a center convex or concave curve in a cross-section view.

14. A microstructure comprising:
a base portion formed on a substrate and having a first cross section;
a separation portion having a second cross section smaller than the first cross section of the base portion and formed on the base portion to extend in a direction away from the substrate; and
a tip portion having a third cross section larger than the second cross section of the separation portion and formed on the separation portion to extend in the direction away from the substrate,
wherein outer surfaces of the base portion, the separation portion, and the tip portion have a curvature continuous in the direction away from the substrate.

15. The microstructure of claim 14, wherein an area of the base portion decreases in the direction away from the substrate,
the second cross section is located at a central portion of the separation portion when viewed in the direction away from the substrate and is the narrowest cross section of cross sections of the separation portion,
the third cross section has a cross section larger than that of one end surface of the separation portion far from the substrate and is the widest cross section of cross sections of the tip portion when viewed in the direction away from the substrate, and
the tip portion is formed in a pointed upper end of which a cross section next to the third cross section of the tip portion is narrowed in the direction away from the substrate.

16. The microstructure of claim 14, wherein the base portion, the separation portion, and the tip portion are integrally formed, or the base portion and the tip portion are formed of different materials.

17. The microstructure of claim 14, wherein a diameter of the first cross section is in a range of 10 to 1,000 µm,
a diameter of the second cross section is in a range of 5 to 500 µm,
an extension length from the substrate to the second end surface is in a range of 50 to 1,000 µm,
a diameter of the third cross section is in a range of 100 to 500 µm, a distance from the third cross section to an end of the tip portion is in a range of 100 to 1,000 µm, and
a total extension length from the substrate to the end of the tip portion is in a range of 200 to 2,000 µm.

18. The microstructure of claim 14, wherein the tip portion include a first volume region located at a first extension length from one end of the separation portion to the third cross section in the direction away from the substrate, a second volume region located at an extension length twice the first extension length from the third cross section, and a third volume region located above the second volume region,
a combined volume of the first volume region and the second volume region is 2 to 1000 times a volume of the third volume region,
the tip portion has a volume of 1.5 to 100 times a volume of the base portion and the separation portion, and
a volume in a height range of an upper 60% in a total height direction from the base portion to the tip portion is in a range of 60 to 90% of a total volume.
